(19) Europäisches Patentamt
European Patent Office
Office européen des brevets

(11) **EP 2 419 442 B1**

(12) **EUROPEAN PATENT SPECIFICATION**

(45) Date of publication and mention of the grant of the patent:
**16.11.2016 Bulletin 2016/46**

(51) Int Cl.:
*A61L 27/22* (2006.01)        *C07K 14/78* (2006.01)
*A61K 38/39* (2006.01)        *A61L 27/52* (2006.01)

(21) Application number: **10723778.6**

(22) Date of filing: **15.04.2010**

(86) International application number:
**PCT/IB2010/051642**

(87) International publication number:
**WO 2010/119420 (21.10.2010 Gazette 2010/42)**

(54) **3D MATRICES OF HUMAN ELASTIN-LIKE POLYPEPTIDES AND METHODS OF PREPARATION THEREOF**

3D MATRIZEN VON MENSCHLICHEN ELASTINÄHNLICHEN POLYPETIDEN UND VERFAHREN ZU IHRER HERSTELLUNG

MATRICE EN 3D DE POLYPEPTIDES DE TYPE ELASTINE HUMAINE ET PROCÉDÉ POUR LEUR PRÉPARATION

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **16.04.2009 IT PD20090092**

(43) Date of publication of application:
**22.02.2012 Bulletin 2012/08**

(73) Proprietor: **Universita' Degli Studi Di Trieste 34127 Trieste (IT)**

(72) Inventor: **BANDIERA, Antonella I-34134 Trieste (IT)**

(74) Representative: **Gervasi, Gemma Notarbartolo & Gervasi S.p.A. Corso di Porta Vittoria 9 20122 Milano (IT)**

(56) References cited:
- **BANDIERA A., SIST P., TERDOSLAVICH M. ET AL.: "Phase transition and particle formation of a Human Elastin-Like polypeptide" BIOENGINEERING CONFERENCE, 2009 IEEE 35TH ANNUAL NORTHEAST, [Online] 3 April 2009 (2009-04-03), - 5 April 2009 (2009-04-05) pages 1-2, XP031460701 ISBN: 978-1-4244-4362-8 Retrieved from the Internet: URL:http://ieeexplore.ieee.org/stamp/stamp .jsp ?arnumber=4967772&isnumber=4967618> [retrieved on 2010-01-18]**
- **BANDIERA ANTONELLA ET AL: "Expression and characterization of human-elastin-repeat-based temperature-responsive protein polymers for biotechnological purposes" BIOTECHNOLOGY AND APPLIED BIOCHEMISTRY, ACADEMIC PRESS, US, vol. 42, no. 3, 1 December 2005 (2005-12-01), pages 247-256, XP009128167 ISSN: 0885-4513**
- **GARCIA YOLANDA ET AL: "In Vitro Characterization of a Collagen Scaffold Enzymatically Cross-Linked with a Tailored Elastin-like Polymer" TISSUE ENGINEERING. PART A, LIEBERT, US, vol. 15, no. 4, 1 April 2009 (2009-04-01), pages 887-899, XP009128247 ISSN: 1937-3341**

EP 2 419 442 B1

- MCHALE MELISSA K ET AL: "Synthesis and in vitro evaluation of enzymatically cross-linked elastin-like polypeptide gels for cartilaginous tissue repair" TISSUE ENGINEERING, vol. 11, no. 11-12, November 2005 (2005-11), pages 1768-1779, XP007911562 ISSN: 1076-3279 cited in the application
- HU BI-HUANG ET AL: "Rational design of transglutaminase substrate peptides for rapid enzymatic formation of hydrogels" JOURNAL OF THE AMERICAN CHEMICAL SOCIETY, AMERICAN CHEMICAL SOCIETY, NEW YORK, USA, vol. 125, no. 47, 26 November 2003 (2003-11-26), pages 14298-14299, XP002421983 ISSN: 0002-7863
- L.A. SETTON, D.L. NETTLES, D.W. LIM, F. GUILAK AND A. CHILKOTI: "Genetically engineered elastin-like polypeptides for cartilage tissue regeneration" OSTEOARTHRITIS AND CARTILAGE, vol. 15, no. Sup.2, 5 September 2007 (2007-09-05), pages B2-B5, XP022221388
- BELLINGHAM CATHERINE M ET AL: "Recombinant human elastin polypeptides self-assemble into biomaterials with elastin-like properties." BIOPOLYMERS, vol. 70, no. 4, December 2003 (2003-12), pages 445-455, XP007911587 ISSN: 0006-3525
- WAGENSEIL JESSICA E ET AL: "New insights into elastic fiber assembly", BIRTH DEFECTS RESEARCH, vol. 81, no. 4, December 2007 (2007-12), pages 229-240, ISSN: 1542-975X

**Description**

**Field of the invention**

The present invention relates to hydrogels or 3D matrices obtained from human elastin-like polypeptides suitably cross-linked by means of enzymatic cross-linking, and to preparation and use in the biomedical field thereof.

**State of the art**

Hydrogel type matrices are currently raising particular interest for their use in biotechnology and in the biomedical field. Structured biomaterials capable of retaining water play a key role in both regenerative medicine and development of *in vitro* cell cultures. The main field of their use is as support for three-dimensional growth of cell populations and in delivery of drugs to activate specific cellular functions.

Within tissues, the natural environment where cells proliferate and develop is organized around the extracellular matrix (ECM) which forms a scaffold. Therefore, one of the key points for cell growth is to attain an appropriate synthetic substitute for the ECM. Although it is extremely difficult to reproduce the ECM, some basic and essential characteristics have been established. Among those, and in addition to the chemical nature of components, of particular importance are three-dimensionality, porosity, biomechanical properties and the ratio of surface area available for cell growth and volume. In fact, these fundamental microstructural properties of the ECM are also important features that allow the flow of nutrients and biological signals and simultaneous elimination of catabolites. Selection of the components used to make the biomaterial is the basis of its final characteristics. Both synthetic and natural polymers are components of choice, especially when creation of porous structures is the goal.

Currently there is great attention for components belonging to the category of biomimetic polymers, which are based on materials normally found in nature and are produced by means of an artificial process. The main advantages of this approach are represented by the reproducibility and standardization of the starting material and elimination of the biological risk related to the delivery of pathogens. Proteins are one of the main representatives of this category, due to well-established recombinant DNA technologies enabling production of synthetic genes and, hence, of their expression products.

Among proteins found in nature, a model for the biomimetic approach is represented by elastin, a typical component of vertebrate tissues characterized and isolated in 1952 from human aorta and equine ligaments (Wise, S.G. and Weiss, A.S., 2009, Int. J. Biochem. Cell Biol., 41: 494-497). Elastin is a major ECM component and the main constituent of elastic fibers which determine tissue elasticity and store energy during the respiratory and cardiac cycle. Elastin, a highly insoluble protein, is formed upon assembly and cross-linking of the soluble precursor tropoelastin, a 60-70kDa protein composed of alternating hydrophobic and lysine-rich hydrophilic domains. Human tropoelastin is encoded by a single gene of 34 exons localized on chromosome 7q11.23. Said gene can give rise to distinct alternative splicing isoforms in addition to the initial translation product, a 72kDa polypeptide (Wise, S.G. 2009, *cit.ref.*).

Elasticity, glass transition and coacervation are fundamental and particularly interesting biophysical properties of elastin. As for elasticity, a generally accepted model posits that the spontaneous return of elastin to the relaxation state, which precedes extension, is of entropic nature and more precisely relates to the hydration cage formed by water molecules in the environment surrounding protein molecules (Vrhovski, B. and Weiss, A.S., 1998, Eur. J. Biochem., 258: 1-18). Coacervation pertains to phase transition due to temperature changes; in the case of elastin, a temperature rise determines the interaction between hydrophobic domains, excluding the surrounding water. As result, self-aggregation of elastin molecules and separation from water occurs. This phase transition is due to the presence of hydrophobic domains characterized by pentapeptide VPGXG repeats which are typical of bovine tropoelastin (ELN elastin [Bos taurus] Gene ID: 280781, GenBank Accession No. NP_786966). Likewise, synthetic and later recombinant (poly)peptides, based on repeats of this motif, retained the above described properties (Urry, D.W., et al., 2002, Philos. Trans. R Soc. Lond. B Biol. Sci. 357: 169-184).

Polymeric proteins known as elastin-like polypeptides (ELP) were originally developed by DW Urry, based on the sequence repeats found in bovine tropoelastin, and were subsequently varied by several authors.

In U.S. Pat. No. 4,474,851, U.S. Pat. No. 4,500,700, U.S. Pat. No. 4,870,055, U.S. Pat. No. 5,250,516, Urry D.W. describes various elastin-derived components comprising tetra- and penta-peptide repeat units derived from the bovine tropoelastin sequence, while U.S. Pat. No. 4,589,882 describes ELP polypeptides comprising tetra- and penta-peptide repeat units derived from the tropoelastin sequence and components capable of generating cross-linked bonds which can involve amino acids, particularly lysine.

Chilkoti, A., Pat. US No. 2005/0255554A1 describes fusion proteins with transition phase properties comprising ELPs composed of tetrapeptide, pentapeptide, esapeptide, octapeptide, nonapeptide sequence repeats. It is proposed their use as fusion proteins in order to exploit their phase transition properties for purification of the whole expression product.

Keeley, F.W. and co-workers focused their attention on the sequence bearing a more regular pattern of the hexapeptidic repeat motif found in human tropoelastin (ELN Elastin [Homo sapiens] Gene ID: 2006, *isoforms* a and d GenBank Accession No. NP_000492.2 and NP_001075223) Val-Ala-Pro-Gly-Val-Gly (VAPGVG), coded by exon 24, for its self-assembly ability (Keeley, F. W., U.S. Pat. No. 2003/0166846A1, Bellingham C. M., et al., 2001, Biochim. Biophys. Acta, 1550: 6-19). In particular, the patent describes self-assembling peptides derived from the hexapeptidic motif of human tropoelastin. It is proposed their use as coating materials for implantable prostheses and as part of preparations for cosmetic use.

Artificial proteins based on hydrophobic elastin motifs also represent a basic material adaptable for the preparation of matrices with characteristics of hydrogel. Several authors have studied properties, characteristics and applications of elastin-like polypeptides organized so as to give rise to three-dimensional supports.

Weiss and co-workers investigated the preparation of hydrogels using both α-elastin extracted from bovine ligament and recombinant human tropoelastin as basic macromolecule.

In the first case, glutaraldehyde and high pressure $CO_2$ environment were used to cross-link bovine ligament α-elastin fragments, thereby obtaining a material with suitable porosity and better texture than the material obtained at ambient pressure (Annabi, N., et al., 2009, Biomaterials, 30, 1-7).

Instead, human recombinant tropoelastin (GenBank entry AAC98394 (gi182020)) was used to prepare hydrogels by use of chemical and physical methods. The use of bis-succinimidyl suberate for irreversible cross-linking of the recombinant protein is described. By this procedure, biomaterials were obtained in the form of sponges and sheets (Mithieux S. M., et al., 2004, Biomaterials, 25: 4921-4927). Regarding production of materials by physical methods, a coalescence of aggregates up to the irreversible self-assembly into a solid and robust hydrogel is obtained by varying appropriately the conditions of pH, temperature and recombinant tropoelastin concentration (Mithieux, S.M. et al., 2009, Biomaterials, 30: 431-435).

Chilkoti and other co-authors (U.S. Pat. No. 2008/0312156A1) also describe hydrogels, to be prepared *in situ* for repair of cartilage tissue defects, which are derived from ELP polypeptides based on the Val-Pro-Gly-Xaa-Gly (VPGXG) pentapeptide motif obtained by cross-linking with chemical agents such as phosphine ligands without amino groups.

These authors have made hydrogels by use of polypeptides of various lengths based on the pentameric repeat pattern of bovine tropoelastin and its variations, in particular at the fourth amino acid of the repeat, -VPGXG- where X stands for any amino acid, except that it is a lysine every 7 or 17 repetitions. The lattice was made by chemical means, using tris-succinimidyl aminotriacetate (Trabbic-Carlson, K., et al., 2003, Biomacromolecules, 4: 572-580), or organophosphoric cross-linking agents lacking amino groups containing hydroxymethylphosphine such as β-[tris (hydroxymethyl) phosphino]-propionic acid capable of reacting with primary and secondary amines producing stable aminomethylphosphines (Lim, D.W., 2007, Biomacromolecules, 8: 1463-1470).

The same author describes hydrogels to encapsulate chondrocytes and prepared by an enzymatic method, in particular by use of a calcium-activated recombinant human tissue transglutaminase produced as cross-linking agent by the authors themselves. The enzymatic reaction is performed in the presence of the cells, using two types of elastin-like polypeptides based on the VPGXG pentapeptide motif, where X can be glutamine in a polypeptide or lysine in the other polypeptide, thus enabling covalent bond formation (McHale, M. K., et al., 2005, Tissue Eng., 11: 1768-1779).

Both W. Chen's group (Lao, U. L., et al., 2007, Biomacromolecules, 8: 3736-3739) and H. Ghandehari's group, which uses also repeat motifs typical of silk (Dinerman, A. A., et al., 2002, Biomaterials, 23: 4203-4210) reported the production of materials with characteristics of hydrogel by use of polypeptides based on the VPGXG pentapeptide motif from bovine tropoelastin.

Lao, U. L., *et al. (cit.ref.)* describe the preparation of physically reversible hydrogels from elastin-like polypeptides based on the VPGXG repeated sequence of bovine tropoelastin with a so-called three-blocks structure, characterized by alternating hydrophobic-hydrophilic-hydrophobic blocks, which imparts gelling ability to solutions at a critical concentration which, according to these authors, is 6% w/v at room temperature. Upon cooling, the gel is dissolved and the polymer returns in solution. The authors propose its use for chelation of metal ions (especially cadmium) in solution.

Cappello and Ghandehari with other co-authors describe SELP polypeptides (Silk-elastin-like protein-based polypeptides) whose structure comprises both the typical repeats of bovine tropoelastin and the repeats typical of the silk protein capable of acquiring an ordered structure. In this way, the macromolecule is composed of amorphous zones (elastin-like) alternating with ordered zones (silk-like), a structure that induces its irreversible gelation in solution by controlling parameters such as concentration, temperature and pH (Dinerman, A. A., *et al., cit. ref.).*

With regard to recombinant polypeptides based on the VAPGVG hexapeptide repeated motif of human tropoelastin, the polypeptides modeled on the basis of human elastin, described by Keelely, have higher relevance. Materials derived by exploiting their self-assembly properties have been described for these polypeptides (Bellingham, C. M., et al., 2003, Biopolymers, 70: 445-55) which are obtained by chemical cross-linking by the agent pyrroloquinoline-quinone that forms bonds at minimal distance. Moreover Keeley and colleagues (Vieth, S. et al., 2007, Biopolymers, 85: 199-206) describe the production of materials with high tensile strength starting from the above described self-assembling polypeptides (Keeley, F. W., U.S. Pat. *cit. ref.).* Preparations in the form of sheets are obtained by cross-linking with pyrroloquinoline-quinone and the naturally occurring agent genipin. Another cross-linking agent used to obtain hydrogels from elastin-like polypeptides is lysine isocyanate, whose reaction products are considered sufficiently tolerable (Srokowski, E.M., et al., 2008, J. Biomater. Sci. Polym. Ed., 19: 785-799).

The group of Rodriguez-Cabello has also designed an elastin-like polypeptide which includes the VPGXG repeat motif of bovine tropoelastin. The authors, together with Pandit's group, have used this polypeptide along with collagen for the preparation of hydrogels (Garcia Y. et al., 2009, Tissue Eng. Part A., 15, 887-899). In particular, cross-linking of the collagen-peptide mixture is carried out enzymatically using a commercial microbial transglutaminase.

The transglutaminase enzyme could be a good candidate as cross-linking agent for these biopolymers, being able to catalyse an acyl-transfer reaction between glutamine and lysine residues. However, according to what is disclosed by Hu Bi-Huang and Messersmith P. (Hu Bi- Huang, Messersmith P., 2003, J. Am. Chem. Soc., 125, 14298-14299) the peptide substrate candidates must have some basic features: i) two or more Gln residues; ii) an hydrophobic amino acid, such as Leu or Phe, adjacent to Gln residue near to C-terminus; and iii) the substitution of Gly residue with a hydrofobic aminoacid, such as Leu, near to the Lys residue on the N-terminus.

Also the inventor in collaboration with other scientists has tried to find out a polypeptide based on human elastin-like repeats to mimic elastin (Bandiera A., et al., 2005, Biotechnol. Appl. Biochem., 42: 247-256) but the polypeptide formed by eigth blocks of seven hexapeptidic repeats has shown a coacervation behavior in near-physiological ionic strength conditions. The coacervation was reversible at temperature of about 38 °C but irreversible over the transition temperature in presence of salt (Bandiera A. et al., Bioengineering Conference, 2009 IEEE 35th Annual Northeast, 03-05 April, 2009).

Nevertheless the problem of having available biocompatible systems that can incorporate cells and maintain their phenotype, also enabling growth and replication, reflects an as yet unmet need.

Therefore, a first aim of the present invention is to develop biocompatible systems, applicable to the field of tissue engineering, for cell culture and encapsulation and able to ensure not only cell survival but also maintenance of phenotypic characteristics, growth and replication.

A further aim is to develop such systems by use of biomimetic polypeptides capable of forming stable and lasting bonds without chemical manipulations or the need for complex manipulations to prepare such systems.

## Summary of the invention

To pursue the above mentioned purposes, the inventors have identified appropriate macromolecules consisting of elastin-like polypeptides derived from the human tropoelastin sequence, and comprising the repeated sequence VAPGVG, and sequences comprising amino acids capable of forming, under appropriate conditions, mutual and stable covalent bonds, thereby creating 3D matrices.

Therefore, in a first aspect, object of the present invention are hydrogels or 3D matrices obtained by transglutaminase enzymatic cross-linking of at least one human elastin-like polypeptide with sequence MRGSHHHHHHG-SAA(AAAAAAKAAAKAAQFGLVPGVGVAPGVGVAPGVGVAP GVGLAPGVGVAPGVGVAPGVGVAPGIAP)$_n$GV (SEQ ID NO:1), where n is an integer between 4 and 12, preferably comprised between 6 and 10 and more preferably n equals 8.

The cross-linking can be obtained between a lysine (K) and a glutamine (Q), comprised in the sequence AAAAAAKAAAKAAQF (SEQ ID NO:3 of the monomer repeat unit (SEQ ID NO:2) within SEQ ID NO:1, in particular at position 7 or 11 and position 14 of SEQ ID NO:3, respectively.

In a second aspect, the invention relates to a method for preparation of these matrices comprising at least the following steps:

- preparing an aqueous solution buffered at pH 8 of the human elastin-like polypeptide having SEQ ID NO:1 at a concentration selected in the range from 3% to 6% w/v;
- treating the aqueous solution of such polypeptides with an aqueous solution of transglutaminase to a concentration of 2 mg/ml, at a temperature comprised between 20°C and 25°C.

The ionic strength of the solution of the polypeptide with SEQ ID NO:1 is preferably equal to 0 and not exceeding 150mM.

Optionally the method further comprises a step of elimination of the buffering agent and of the transglutaminase enzyme by diffusion from the hydrogel upon its immersion in an aqueous medium such as water, saline or culture medium.

By use of the above indicated preparation method, cross-linked matrices, characterized by values of the ratio between stretching (v) of the C-N bond (at 1090-1045 cm$^{-1}$) and stretching (v) of the C-H bond (at 2940-2840 cm$^{-1}$) (v (C-N)/v (C-H)) comprised between 0.031 and 0.020, are obtained with the polypeptide SEQ ID NO: 1 (n = 8).

Such 3D matrices are also characterized by:

- a storage module (E ') comprised between the minimum value of $1 \times 10^{-4}$ and a maximum value of $3.5 \times 10^{-3}$ MPa

for matrices prepared with peptide concentrations comprised between 3% and 6% w/v;

- a loss factor (tan$\delta$ = E''/ E ') comprised between 0.002 and 0.6 for matrices prepared with peptide concentrations comprised between 3% and 6% w/v

measured at 37°C in aqueous environment.

The hydrogels or 3D matrices thus obtained can be used both as a flat substratum for seeding and growing cells and as 3D matrix for encapsulating and growing cells.

In the latter case, for encapsulation of cells in the hydrogel of the invention, cells can be added to buffered aqueous solutions of polypeptides with SEQ ID NO:1 prior to the enzymatic cross-linking step; in this case the ionic strength of the polypeptide solution is physiological due to addition of 150mM NaCl. Subsequently, transglutaminase is added and the whole is deposited at the site suitable for cell culture and immersed in culture medium.

One aspect of the present invention relates, in fact, to realization of supports for cell cultures aimed at reproducing a three-dimensional environment for growth that is more similar to the natural environment compared to routinely used two-dimensional supports.

Therefore, the use of such hydrogels or 3D matrices for growing cells to be used in biomedical research, for the purpose of both research and tissue engineering, is according to this latter aspect of the present invention.

A further aspect of the present invention is represented by the possibility of incorporating into matrices, by use of the method described and regardless of whether or not they include cells, other compounds such as pharmacologically or biologically active molecules. Depending on the nature of the compound, this can be:

- merely retained in (and thus concentrated inside) the liquid phase permeating the matrix, resulting in the release of the compound as such following its own kinetics;
- effectively incorporated, thus becoming permanently part of the matrix.

Purposes and benefits of three-dimensional matrices of the present invention will be better understood in the following detailed description which includes, without being limited to them, examples of hydrogels obtained from polypeptides with SEQ ID NO:1 and their physico-chemical characterization as well as biological compatibility with cells isolated and encapsulated within them.

**Brief description of the figures**

**Figure 1.** The figure shows the phase transition profile, monitored by turbidimetry, of a 0.2% solution of the polypeptide with SEQ ID NO:1 (n=8) (hereafter indicated as HELP$_8$) in 10mM TRIS/HCl buffer at pH 7.5 (A) and pH 8 (B).

**Figure 2.** The figure shows the variation of phase transition temperature as a function of concentration of the HELP$_8$ polypeptide in solution containing 10mM TRIS/HCl pH 8 ($\bigcirc$) or 10mM TRIS/HCl pH 8 + 150mM NaCl ($\bullet$).

**Figure 3.** The figure shows the scanning electron microscope (SEM) analysis of 3D matrices containing 5% HELP$_8$, obtained by enzymatic cross-linking with transglutaminase, in the absence (A) and in presence of 150 mm NaCl (B).

**Figure 4.** The figure shows the electrophoretic analysis of samples of 5% HELP$_8$ in 10mM Tris/HCl pH 8, treated with transglutaminase and taken at different times. (A) shows the result of the experiment carried out at room temperature, (B) the same experiment carried out at 37°C.

**Figure 5.** The figure shows SEM analyses of the cross sections of 3D matrices made with 3% (A), 5% (B) and 6% (C) HELP$_8$, highlighting their internal structure. 500x magnification.

**Figure 6.** The figure shows the performance of the storage module as a function of frequency over 11 stress cycles to which samples of 3D matrices, obtained at 5% HELP$_8$ concentration, were subjected (A) and the mean values and standard deviation of the storage module (E') of the same samples on cycle 1, 6 and 11 (B).

**Figure 7.** The figure shows the performance of the storage module as a function of frequency over 11 stress cycles to which the samples of 3D matrices obtained at 6% HELP$_8$ concentration were subjected.

**Figure 8.** The figure shows mean values and standard deviation of the storage module (E') for samples of 3D matrices obtained with 6% HELP$_8$ concentration on cycle 1, 6 and 11 (A) and mean values and standard deviation of the loss factor (tan$\delta$ = E''/E') of the same samples on cycle 1, 6 and 11 (B).

**Figure 9.** The figure shows mean values and standard deviation of the values expressing water absorption and subsequent degradation for samples of 3D matrices obtained with HELP$_8$ concentrations of 5% ($\bullet$) and 6% ($\blacksquare$).

**Figure 10.** The figure shows mean values and standard deviation of the values expressing water absorption in 3D matrices, obtained with 5% HELP$_8$ concentration, within 400 minutes (A) and up to 1500 minutes (B).

**Figure 11.** The figure shows mean values and standard deviation of the values expressing water absorption in 3D matrices, obtained with 6% HELP$_8$ concentration, within 12 minutes (A) and mean values and standard deviation of the values expressing water absorption and subsequent degradation for the same samples up to 1500 minutes (B).

**Figure 12.** The figure shows the time course of growth of the cell line HepG2 on a standard treated plastic support (A) and on a 3D matrix made with 5% HELP$_8$ (B) at different times after cell seeding.

**Figure 13.** The figure shows an example of proliferation assay performed with HepG2 cells seeded on standard treated plastic and on a 3D matrix made with 5% HELP$_8$, over a period of 10 days (240 hours).

**Figure 14.** The figure shows a colony of HepG2 cells that was transferred to standard treated plastic after proliferation for one week on a 3D matrix made with 5% HELP$_8$ (A) and the same colony after 12 additional days of culture (B).

**Figure 15.** The figure shows MCF-7 cells one week after seeding on standard treated plastic (A) and on a 3D matrix made with 5% HELP$_8$ (B).

**Figure 16.** The figure shows MCF-7 cells 24 hours after encapsulation (A) and 168 hours after encapsulation (B) in a matrix made with 3% HELP$_8$.

**Detailed description of the invention.**

*Definitions*

The standard IUPAC nomenclature or the single letter code was used to represent amino acids composing the sequences.

The definition elastin refers to the naturally organized protein derived from tropoelastin. The definition human tropoelastin refers in particular to the isoforms a and d (GenBank Accession No. NP_000492.2 and NP_001075223) which are the products expressed from the ELN gene (GenBank, Gene ID: 2006) containing the following repeated sequence: -AAAAAKAAAKAAQFGLVPGVGVAPGVGVAPGVGVAPGVGLAPGVGVAPGV GVAPGVGVAPGI-.

The term ELP (elastin-like polypeptide) is hereinafter used to refer broadly to elastin-like polypeptides consisting of polypeptides comprising repeated sequences of elastin and tropoelastin having coacervation characteristics.

HELP (Human-Elastin-like polypeptide) is hereinafter used to indicate human-like elastin polypeptides on which hydrogels or 3D matrices of the invention are based. Said polypeptides are derived from the gene coding the artificial protein based on the repeated VAPGVG hexapeptide motif of human elastin as described by Bandiera A., et al., 2005, Biotechnol. Appl. Biochem., 42: 247-256. The sequence of HELP polypeptides is as follows: MRGSHHHHHHG-SAA(AAAAAAKAAAKAAQFGLVPGVGVAPGVGVAPGVGVAP GVGLAPGVGVAPGVGVAPGVGVAPGIAP)$_n$GV (SEQ ID NO:1) where n is an integer between 4 and 12, preferably between 6 and 10 and more preferably is 8.

In said polypeptides with SEQ ID NO:1, the sequence AAAAAAKAAAKAAQFGLVPGVGVAPGVGVAPGVGVAPGVGLAPGVGVAPGVG VAPGVGVAPGIAP (SEQ ID NO:2) is the repeated monomer unit containing both the cross-linking domain and the domain conferring the typical properties of ELP polypeptides. If the monomer unit with SEQ ID NO:2 is repeated 8 times, the polypeptide is identified as polypeptide SEQ ID NO:1 (n=8) or HELP$_8$.

In particular, the sequence AAAAAAKAAAKAAQF (SEQ ID NO:3) of HELP polypeptides is the domain (the so-called cross-linking domain), where the cross-linking reaction takes place, since the amino acids capable of forming stable covalent bonds by a catalyzed reaction are one of the two lysines (K) in position 7 or 11, according to SEQ ID NO: 3, present in one cross-linking domain of HELP polypeptides, and the glutamine (Q) in position 14 according to SEQ ID NO:3 of the other cross-linking domain of HELP polypeptides. Said bonds can be formed between cross-linking domains of distinct molecules or intrachain between cross-linking domains of the same polypeptide molecule. Instead, elastin-like characteristics of the polypeptides are conferred by the repeated sequence region GLVPGVGVAPGVGVAPGVGVAPGVGLAPGVGVAPGVGVAPGVGVAPGIAP (SEQ ID NO:4), comprising the repeated hexapeptide motif VAPGVG (SEQ ID NO:5) which is typical of human elastin.

Hydrogel or hydrogels are semi-solid hydrated structures able to maintain size and shape when subjected to deformation.

3D or three dimensional matrices are solid (not hydrated) or semi-solid (hydrated) structures capable of maintaining size and shape when they are not subjected to deformation. Therefore the terms hydrogels or 3D matrices, or simply matrices, must be considered equivalent, since essentially they both refer to lattices that are formed by HELP polypeptides with SEQ ID NO:1 via enzymatic cross-linking.

For encapsulation or microencapsulation it is meant a process involving the inclusion, within solid or semisolid hydrogels or 3D matrices, of biological material or other materials.

*Description*

The invention relates to the formation, by enzymatic cross-linking, of 3-D matrices based on elastin-like HELP polypeptides with SEQ ID NO:1 having specific features with respect to physical properties, in particular structural and mechanical properties, and capable of rendering said matrices suitable for use preferably as cellular support, maintaining cellular viability, phenotype and proliferative capacity.

As already widely described in the section on the State of the art, several methods are known for the preparation from ELP polypeptides of hydrogels obtained by forming a network between polypeptides or between chain segments of the same polypeptide, in particular at the level of cross-linking domains containing lysine and glutamine (see for example Vrhovski, B. and Weiss, A.S., 1998, *cit. ref.*). Formation of such hydrogels is essentially achieved by phasing the polypeptide chains at the level of these domains, so that lysine and glutamine are favourably positioned for covalent bond formation. In particular, one of the enzymes able to catalyze formation of such bond is transglutaminase, whose reaction catalyzed in aqueous environment leads to formation of a ε-(γ-glutamyl)lysine bridge according to the following scheme:

$$Gln-(CH_2)_2-CONH_2 + {}_2HN-(CH_2)_4-Lys \rightarrow Gln-(CH_2)2-COHN-(CH_2)_4-Lys + NH_3$$

Considering the primary sequence of HELP polypeptides with SEQ ID NO:1, it is noted that both lysine (K) and glutamine (Q) are present in the sequence AAAAAAKAAAKAAQF (SEQ ID NO:3) of the repeated monomer unit (SEQ ID NO:2) and that such sequence may therefore function as cross-linking domain in a HELP polypeptide, since a similar reaction leading to formation of a ε-(γ-glutamyl)lysine bridge may occur between a glutamine of a cross-linking domain AAAAAAKAAAKAAQF (SEQ ID NO:3) and a lysine of a second cross-linking domain AAAAAAKAAAKAAQF (SEQ ID NO:3) of another HELP polypeptide with SEQ ID NO:1.

Nevertheless, although covalent bond formation involving a lysine and a glutamine of two different cross-linking domains is plausible, as well it is plausible, during the cross-linking process, that intrachain bonds are formed within the same polypeptide to some extent, such cross-linking processes do not necessarily lead to formation of hydrogels with appropriate biophysical characteristics for the intended purpose, since the cross-linking reaction may result in structurally inhomogeneous matrices, ultimately affecting mechanical properties. It is especially important to ensure homogeneity in the appearance of the matrix, at the macroscopic level, and of structure and porosity. In addition to altering the shape of the matrix obtained, more or less compacted areas cause discontinuities in the distribution of the aqueous solution permeating the matrix when it is substantially in the form of hydrogel.

Creation of a suitably porous "sponge-like" structure, an environment uniformly permeable to the aqueous phase in all areas, must be associated with an appropriate degree of elasticity of the structure itself which, upon mechanical challenge, must return almost completely to the initial state without significant residual deformation. In order to obtain 3D matrices with appropriate characteristics, i.e. mainly as homogeneous as possible structure with respect to both matrix global shape and micro-structural properties, the approach of enzymatic cross-linking, carried out under appropriate reaction conditions, has been pursued in order to avoid the use of chemicals that are potentially toxic and harmful or otherwise unsuitable for the intended use.

The degree of cross-linking depends on many variables, mainly: i) polypeptide concentration; ii) pH and ionic strength of solutions; iii) reaction temperature. In addition variables come into play which affect the degree of aggregation of the polymer, such as: iv) the amount of enzyme used and v) reaction time.

For the purpose of the present invention, conditions to prepare, from HELP polypeptides, matrices with three-dimensionality, porosity, mechanical properties and a surface/volume ratio appropriate to support cellular growth are as follows:

- concentration of HELP polypeptides with SEQ ID NO:1 in the range between 3% and 6% w/v;
- solvent: aqueous solutions buffered at pH 8;
- enzyme: 60mg/ml transglutaminase solution, preferably commercial of microbial origin, used at 1:30 dilution (=2mg/ml);
- ionic strength: comprised between 0 and 150 mm NaCl;
- temperature: 20-25°C.

If 3D matrices according to the invention are used as carriers for encapsulation of cells and/or biologically or pharmacologically active substances, said cells and/or active substances are further added to the aqueous solutions of polypeptides with SEQ ID NO:1.

As will be clearer in the experimental section below, such conditions proved essential in order to obtain hydrogels or 3D matrices with the desired biophysical characteristics and appropriate to ensure that such matrices can be used as support for cellular growth and for encapsulation of cells.

In fact, with respect to features of hydrogels obtained from HELP polypeptides, in particular when n=8 in SEQ ID NO:1, it was verified that a matrix with suitable consistency to support cellular growth and capable of elastic recovery, can be obtained with HELP solutions in the concentration range between 3% and 6% (w/v). Use of concentrations lower than 3% fails to achieve a matrix with a texture that can be manipulated and to maintain the three-dimensional shape, because the lattice tends to collapse on itself, thereby excluding water. Above 6%, the matrices that are obtained show a three-dimensional structure that is too compact and less homogeneous.

Solutions made with a single artificial protein with SEQ ID NO: 1 (n=8) in bidistilled water were found to have a pH comprised between 7.2 and 7.3, hence quite close to physiological. However, it was observed that phase transition of the polypeptide with SEQ ID NO:1 (n = 8) is a process showing a proper critical specificity. Indeed, the turbidimetric analysis of dilute polypeptide solutions highlighted the fact that even slight pH variations can result in particularly stable inhomogeneous aggregation that is difficult to reverse in solution, as occurs using a buffer at pH 7.5. When the solution is stabilized at pH 8 with Tris/HCl, the polypeptide with SEQ ID NO:1 (n=8) shows the desired elastin-like type of behavior, with a rather fast phase transition and a good reversibility of this process, as shown in the experimental part.

Moreover, the presence of buffer favours a higher reproducibility avoiding any medium acidification due to the action of the enzyme.

In addition to pH, the ionic strength is particularly significant in the cross-linking reaction with HELP polypeptides with SEQ ID NO:1.

For preparation of aqueous solutions of polypeptides with SEQ ID NO:1 it is preferable to use a 10 mM Tris/HCl buffer solution with ionic strength equal to 0 or maximum up to 150mM, where the salt is NaCl. These conditions avoid the interference with self-assembly of polypeptides in the starting solution, producing a faster cross-linking reaction. Therefore aqueous solutions of HELP buffered with Tris/HCl at pH 8, and without added salt, are preferable for the preparation of matrices which, at a later time, are seeded with cells.

In the case providing for encapsulation of cells concurrent with preparation of the matrix, use of 150 mM NaCl solutions is preferable in order to avoid excessive cellular stress and consequent loss of viability.

The concentration of 2mg/ml transglutaminase was selected because it was observed experimentally that, at this concentration of enzyme, the reaction reached completion within 15'-20' for more concentrated $HELP_8$ solutions (5% and 6%).

Concerning the temperature, the most favourable conditions are represented by temperatures comprised between 20°C and 25°C, although higher temperatures (28°C to 37°C) are optimal for enzyme action.

In conclusion, the experimental results highlighted the possibility to effectively obtain materials with the desired characteristics of stable hydrogel, starting from aqueous solutions of HELP with SEQ ID NO:1. Moreover, it was also evident that differences in gelification conditions affect properties and structure of the material obtained, at both macroscopic and microscopic levels.

Therefore, hydrogels or 3D matrices according to the invention can be prepared by mixing, at room temperature, aqueous solutions buffered at pH 8 with Tris/HCl of at least one polypeptide with SEQ ID NO:1 at concentrations comprised between 3% and 6% w/v, at ionic strength comprised between 0 and 150mm, together with aqueous solutions of transglutaminase at a concentration of 2mg/ml. The two aqueous solutions respectively containing the HELP polypeptide with SEQ ID NO:1 and transglutaminase are quickly mixed together and then the whole resulting solution is placed in a suitable container and left at room temperature for at least 30 minutes.

Once prepared, the matrix can be stored in aqueous solution (water, saline, culture medium, etc.). Typically, this step favours diffusion out of the matrix of the enzyme and of the other agents used in cross-linking reactions, as for instance buffering agents, and their easy removal by replacement of the aqueous medium. Although the presence of both enzyme and buffering agent does not affect the properties of the hydrogel or of the cells seeded on top or encapsulated, these components may be removed from the so obtained hydrogels when they need to be used as support for cell growth. In this case, to prevent any unpredictable cellular effect due to the presence of the enzyme, the matrix can be left for 3 to 18 hours in physiological solution or culture medium prior to seeding, in order to favour diffusion and, hence, removal of the enzyme. As an alternative the matrix can be stored in dried form for a long-term storage, after lyophilization.

The preparation method of the invention has proved appropriate with respect to formation of a covalent ε-(γ-glutamyl)lysine bond by transglutaminase catalyzed reaction and production of hydrogels or 3D matrices with appropriate three-dimensionality and dynamic-mechanical behaviour.

Indeed, by Fourier transform infrared spectroscopy in attenuated total reflection mode (ATR-FTIR) done with the polypeptide with SEQ ID NO:1 (n = 8), it was possible to verify that the degree of cross-linking, measured as stretching ratio (ν) between the C-N bond (at 1090-1045 cm$^{-1}$) and stretching (ν) of the C-H bond (at 2940-2840 cm$^{-1}$) (ν (C-N)/ν (C-H)), shows a linear profile for different concentrations of the $HELP_8$ polypeptide with SEQ ID NO:1 and that the 3D matrices obtained are characterized by ν (C-N)/ν (C-H) values comprised between 0.031 and 0.020.

Said matrices prepared with aqueous solutions at $HELP_8$ concentrations comprised between 3 and 6%, have also been shown to have: a) a storage module (E') comprised between the minimum value of $1 \times 10^{-4}$ and a maximum value of $3.5 \times 10^{-3}$ MPa; b) a loss factor (tanδ = E''/E') comprised between 0.002 and 0.6 measured at 37°C in aqueous environment.

The behaviour shown by compression using dynamic-mechanical analysis of hydrogels containing a concentration of $HELP_8$ comprised between 3% and 6% was found to be strongly influenced by the structural organization. In particular, while for matrices containing 6% $HELP_8$ the contribution of the elastic component increases with stress frequency, those with a lower HELP concentration showed a contribution of the constant elastic component which is independent from stress frequency. This different mechanical behaviour is also reflected in a different ability and velocity of recovery of shape following withdrawal of the applied stress.

Hydrogels or 3D matrices according to the invention proved suitable for use as cell culture support, as they are capable of ensuring survival and phenotypic characteristics of the cells they support, as shown in the experimental part. Those are envisaged as supports especially suitable for growth of certain types of cells that physiologically adhere to elastin-containing structures, such as fibroblasts, smooth muscle cells, chondrocytes, keratinocytes, osteocytes and also stem cells.

Cells seeded on this type of support can proliferate on its surface, but may possibly penetrate and infiltrate the support and proliferate inside. Therefore, substantially, the method for seeding cells onto 3D matrices prepared with polypeptides with SEQ ID NO:1, rather than encapsulating cells during preparation of such matrices, depends on operational requirements and on the type of use provided for the cell culture.

On this basis, the use of such supports can thus be envisaged:

- *in vitro,* for biotechnological and research purposes, such as mimetics of the physiological cell environment which are alternative to traditionally used materials;
- *in vivo,* as suitable supports for infiltration of endogenous cells for tissue regeneration/restoration/augmentation or as supports suitable for delivery of exogenous cells for the same purposes.

Experimental part

*Example 1: preparation of HELP$_8$ polypeptide with SEQ ID NO:1 (n = 8)*

The polypeptide was prepared as described by Bandiera A., et al., 2005, Biotechnol. Appl. Biochem., 42: 247-256. The synthetic gene encoding HELP$_8$ was inserted into vector pQE-8 (Qiagen) and the resulting vector was checked by standard sequencing. HELP is expressed in the E. *coli* bacterial strain NEB Express *I$^q$* co-transformed with the pLysS vector. The expression is induced with 0.1 mM IPTG and the bacteria are typically collected after 4 hours. Cells are lysed under native conditions (20mM Tris/HCl pH 8, 100mM NaCl, 0.1 mM EDTA, 10 mM 2-mercaptoethanol, 0.1 mM PMSF, 0.1% (v/v) Tween 20) by sonication. After cold centrifugation (5°C), the HELP$_8$ polypeptide is precipitated from the supernatant brought to 1.5M NaCl raising temperature (42°C), taking advantage of its phase transition properties. The precipitate is collected by centrifugation at 37 ° C and resuspended in cold water. Three of such cycles are typically performed. After the final resuspension in water, the HELP containing solution is essicated by lyophilization.

*Example 2: preparation of 3D matrices of the polypeptide HELP$_8$ with SEQ ID NO:1 (n = 8)*

Typically, a 50mg/ml HELP$_8$ solution is prepared starting from the lyophilized polypeptide which is weighed, placed in a suitable vessel and resuspended in an appropriate volume of buffered solution 10 mM Tris/HCl pH 8 in order to obtain the desired concentration of HELP$_8$; the solution is kept in the cold (on ice) for 15 minutes. The solution is collected, left to equilibrate to room temperature (typically 20-25°C) and then supplemented with the enzyme solution. The enzyme used is a commercial bacterial transglutaminase (TGase) (Bacterial Transglutaminase, N-Zyme Biotec GmbH) used to prepare a 60mg/ml concentrated aqueous solution, which is used at 1:30 dilution for the reaction (therefore the working concentration of the enzyme is 2mg/ml).

Immediately after addition of the enzyme, all the solution is quickly mixed, poured in the mold and left at room temperature for at least 30'. The so obtained matrix can be stored in aqueous solution (water, saline, culture medium, etc.) or can be kept dried after lyophilization.

*Example 3: characterization of conditions for the cross-linking reaction with HELP$_8$ polypeptide with SEQ ID NO:1 (n = 8) for preparation of 3D matrices*

*A. pH*

The phase transition of the polypeptide was studied by turbidimetry in HELP$_8$ aqueous solutions not treated with transglutaminase, monitoring the change in absorbance of the solution at 350nm using a Varian DMS80 UV-vis spectrophotometer equipped with a thermostatic cell at a heating rate of 1° C/min. A concentration of 0.2% HELP$_8$ was used for the experiment in order to prevent any artefact due to non-limpid solutions.

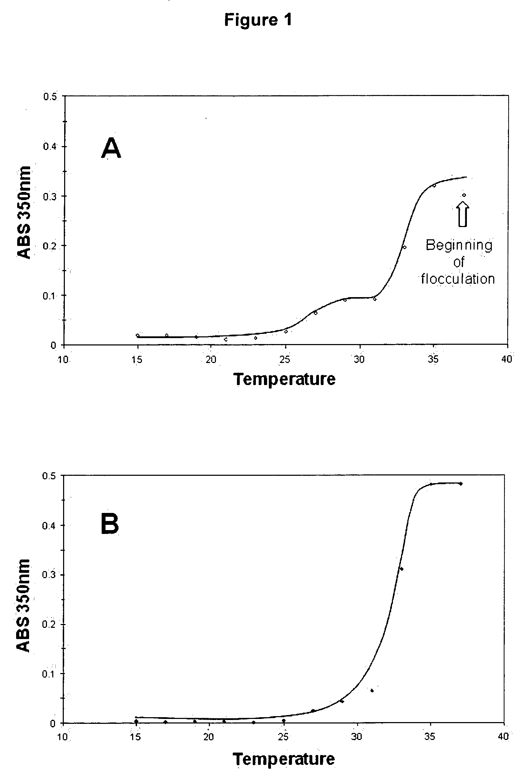

Figure 1 shows phase transition profiles for 0.2% HELP$_8$ solutions:

- at pH 7.5 (Figure 1, A), a non-uniform process is observed in which aggregation begins gradually in a small fraction of the polypeptide and then proceeds more rapidly until completion with formation of macroscopic aggregates. This is probably due to inhomogeneous aggregation although very stable in solution, since it is also difficult to reverse;
- at pH 8 (Figure 1, B), the profile was more similar to those reported in the literature for ELP polymers.

*B. Ionic strength*

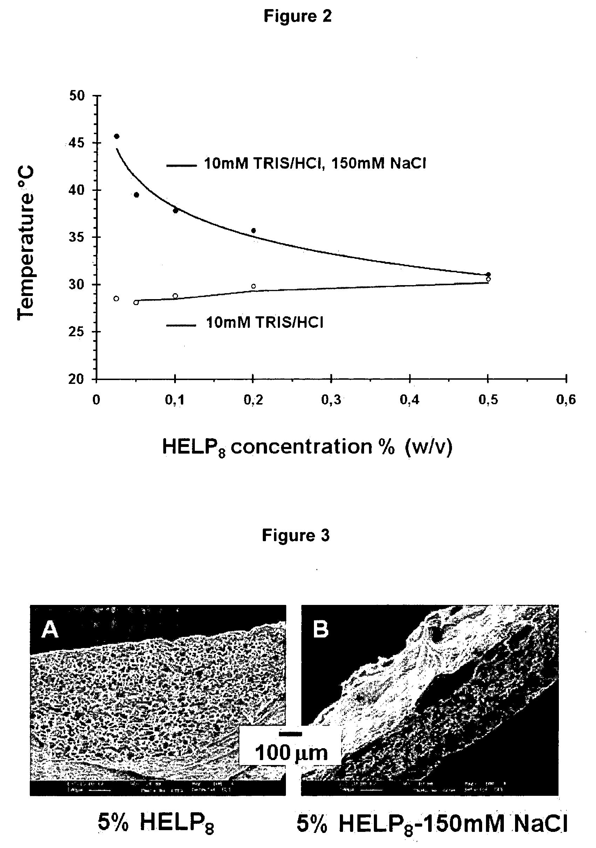

Figure 2 shows the semi-transition temperature of $HELP_8$ as function on its concentration in aqueous solutions not treated with transglutaminase, i.e. the temperature at which half of the polypeptide is found in an aggregated state, as determined by turbidimetry as a function of polymer concentration under conditions of absence (○) and presence (●) of 150mM NaCl, respectively. These measurements showed an abnormal behaviour compared to that described for ELP polymers in general. For the latter, a lower temperature phase transition of the macromolecule corresponds to the increase of ionic strength (Luan, C.H., et al., *cit. ref*). An unexpected behaviour was observed of low concentrated HELP solutions, between 0.05% and 0.5%, under ionic strength conditions from 0 to 150mM, since the addition of NaCl results in an increase rather than a decrease of the transition temperature of the polypeptide, as shown by the experimental results (Figure 2). This probably indicates a specific self-assembling ability of polypeptide chains that confers a particular order to the structure.

Indeed, it has been found experimentally that the addition of 150mM NaCl during matrix preparation for transglutaminase-mediated cross-linking gives rise to matrices with much less homogeneous internal structure, as detected by scanning electron microscope images (Figure 3) showing the appearance of the internal structure of 5% $HELP_8$ matrices cross-linked at room temperature, in the absence (A) or presence (B) of 150 mm NaCl.

*C. Enzyme concentration*

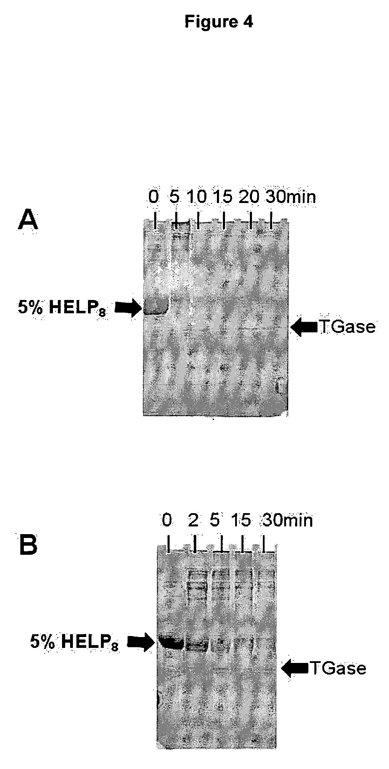

In figure 4, SDS-PAGE electrophoresis was used to follow the action of transglutaminase on 5% $HELP_8$ in Tris/HCl 10mM pH 8 at room temperature, blocking its action at various times by the addition of denaturing solvent samples for electrophoresis (A). Chains initially in a free state are trapped, as the reaction proceeds, in lattices of higher molecular weight which can no longer migrate in the gel. The presence of transglutaminase in all wells shows that any free protein molecules would be also detected and that the disappearance of $HELP_8$ is not due to methodological artefacts but is rather due to its incorporation in the developing lattice. This process is almost complete at room temperature within 5', as shown in Figure 4 (A), and is definitely complete after 10'.

*D. Temperature*

The room temperature (20-25°C) proved the most favourable condition for matrix preparation, as shown in Figure 4. In particular, (B) shows the result of an experiment identical to the previous experiment, but conducted at 37°C rather than room temperature. It can be observed that, for an equal time, there is more polypeptide left free if the enzyme is allowed to act at 37°C. This clearly indicates that cross-linking at this temperature is less effective due to the induction of phase separation of the polypeptide that prevents enzyme access to cross-linking sites.

*Example 4: Characterization by scanning electron microscopy of the structure of solid matrices obtained by cross-linking of $HELP_8$ polypeptide with SEQ ID NO:1 (n = 8)*

For this analysis, matrices were frozen at -20°C immediately after preparation and dried by lyophilization. Samples thus obtained were cut with a razor blade, mounted on appropriate supports for microscopy and covered by a metal film. The analysis was performed with a Leica Stereoscan 430i integrated system microscope for scanning electron microscopy.

Matrices were prepared at 3, 5 and 6% $HELP_8$ under conditions described in example 2.

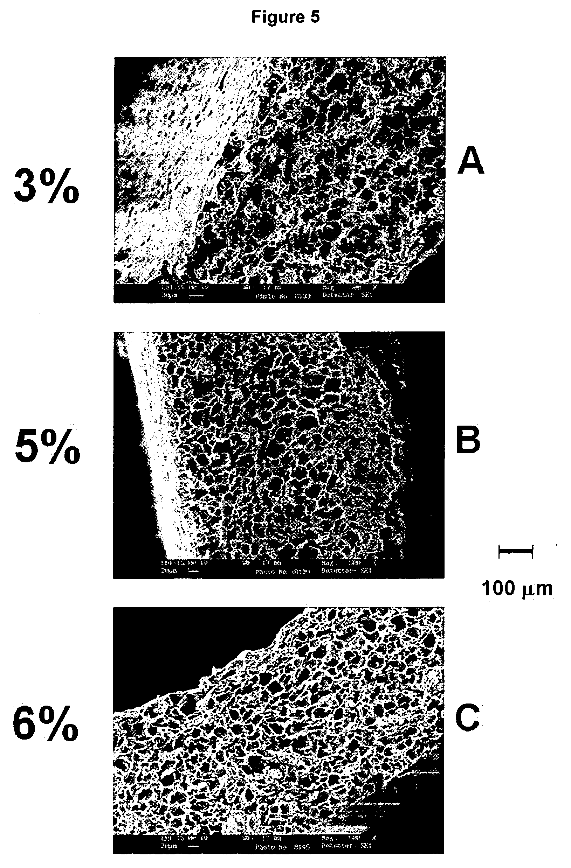

In all three cases, a material is obtained which shows homogeneous structure, spongy appearance, occupies the whole volume of the mold in which cross-linking has been performed and shows pores of various sizes, however in the order of micrometers. Photomicrographs in figure 5 show different magnifications of the structures detected. According to this analysis, narrower cavities and progressive thickening of surrounding walls correlated with the increased percentage of peptide.

*Example 5: characterization by compression using dynamic-mechanical analysis (DMA) of hydrogels obtained by cross-linking the $HELP_8$ polypeptide with SEQ ID NO:1 (n = 8)*

To evaluate dynamic-mechanical properties of $HELP_8$ matrices obtained by cross-linking with transglutaminase, the analysis was performed using a DMA analyzer (Dynamic Mechanical Analyzer, Model 2980, TA Instruments) in compression mode at 37°C with a frequency ramp comprised from 0.5 and 5 Hz. Matrices with 5% and 6% $HELP_8$ concentrations were prepared as described in example 2. For each matrix tests were performed in triplicate.

Samples under compression tests at 37°C were maintained in a water environment for all the test; an isotherm was first applied for 5 minutes at 37°C, followed by a compressive stress with an oscillation amplitude of 20 $\mu$m, 0.05 N preload with a frequency ramp between 0.5 and 5 Hz (0.5, 1, 2, 3, 4, 5 Hz); 11 cycles were performed.

Matrices show a storage modulus (E') between the minimum value of $7 \times 10^{-4}$ and a maximum value of $3.5 \times 10^{-3}$ MPa and a loss factor value (tan$\delta$, given by the ratio E "/E') between 0.002 and 0.6.

*A. 5% $HELP_8$ matrices*

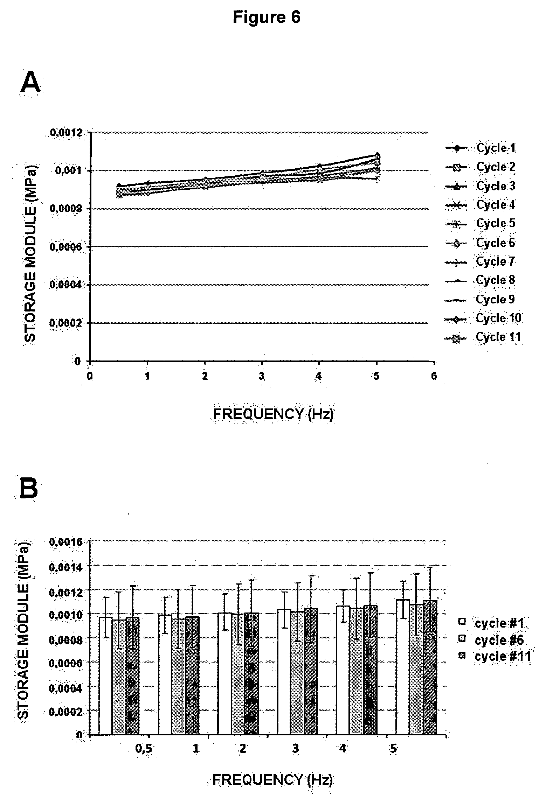

Figure 6A shows the evolution of the storage module (E') as a function of frequency over 11 stress cycles applied to the samples. With increasing frequency analysis and number of cycles applied to the sample, it is noted that the value of the storage module E' remains in a limited range of values, showing that elastic contribution remains constant during the test.

This is also confirmed by the values of the storage module at the first, sixth and eleventh stress cycle (no significant difference). Figure 6B shows mean values and standard deviation of the storage module of 5% HELP samples for cycle 1, 6 and 11.

*B. 6% $HELP_8$ matrices*

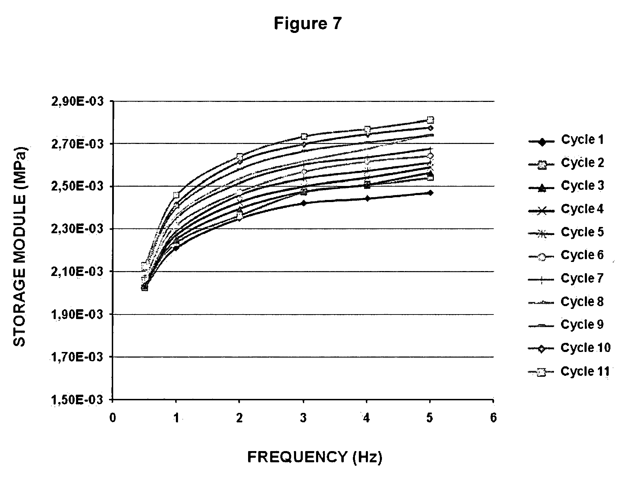

The same dynamic-mechanical analyses were carried out for samples with 6% $HELP_8$ concentration. Figure 7 shows the profile of the storage module (E') as a function of frequency over 11 stress cycles. With increasing frequency analysis and number of cycles applied to the sample, it is noted that the value of the storage module increases, indicating a higher contribution of the elastic component with increasing stress frequency.

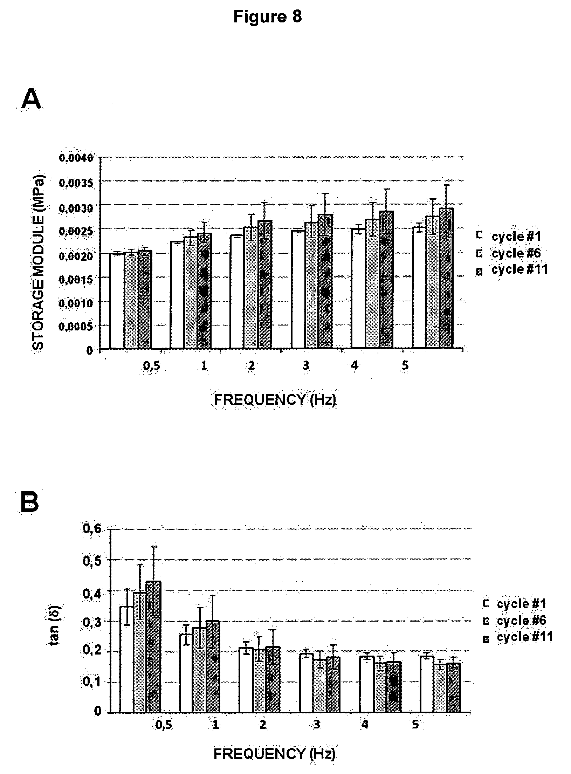

Figure 8 shows mean values and standard deviation of storage module (E') (A) and loss factor (tan$\delta$) (B) of samples containing 6% $HELP_8$ concentration at cycle 1, 6 and 11. It can be noted that the value of the storage module increases with increasing stress frequency at the first stress cycle, while no significant differences are found between the sixth and eleventh cycle at any stress frequency.

*Example 6: Swelling (water uptake) and weight loss characterization of 3D matrices obtained by cross-linking of the $HELP_8$ polypeptide with SEQ ID NO:1 (n = 8)*

When tested for swelling in distilled water at room temperature, dehydrated $HELP_8$ matrices cross-linked by transglutaminase show the maximum degree of swelling (water uptake, WU%), calculated as in formula (1), between 1000% and 3000%.

At the absorption plateau value, matrices show a weight loss that is dependent on the used HELP concentration:

$$W.U.\% = \frac{P_w - P_d}{P_d} \cdot 100 \qquad \text{formula (1)}$$

where:

- $W.U.\%$ = percent swelling (*water uptake*)
- $P_w$ = weight of swollen specimen
- $P_d$ = weight of dried specimen.

Water uptake and degradation tests were performed on matrices obtained with 5% and 6% HELP concentrations. Tests were performed in triplicate, in distilled water, at room temperature, by weighing the samples at different test time points (5, 10, 15, 30 minutes, 1, 2, 3, 4, 6, 8 and 24 hours). At each time point, the weight variation, was calculated with respect to the dry weight at the starting time, by using formula (1),

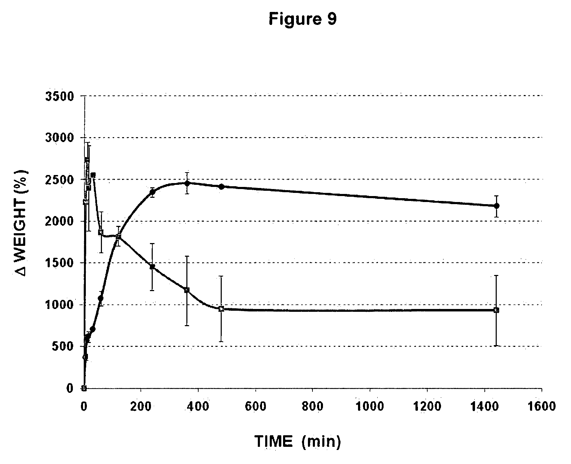

Figure 9 shows the profiles of water absorption and subsequent degradation over time for the two considered $HELP_8$ matrices. Mean values and standard deviation are reported for water absorption and subsequent degradation for matrices obtained with $HELP_8$ concentrations of 5% (●) and 6% (■).

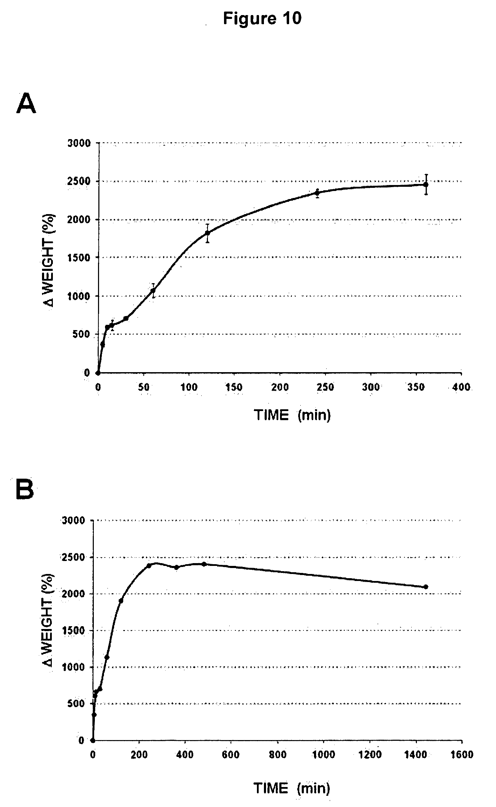

In Figure 10, A shows mean values and standard deviation of water absorption values of matrices obtained with 5% $HELP_8$ concentration, relative to the first part of the curve (within 400 min), regarding the attainment of the maximum absorption, occurred after 360 minutes. After this time point and the following attainment of the maximum absorption, there is a slight weight loss probably due to a leak of material cohesion, as shown in B.

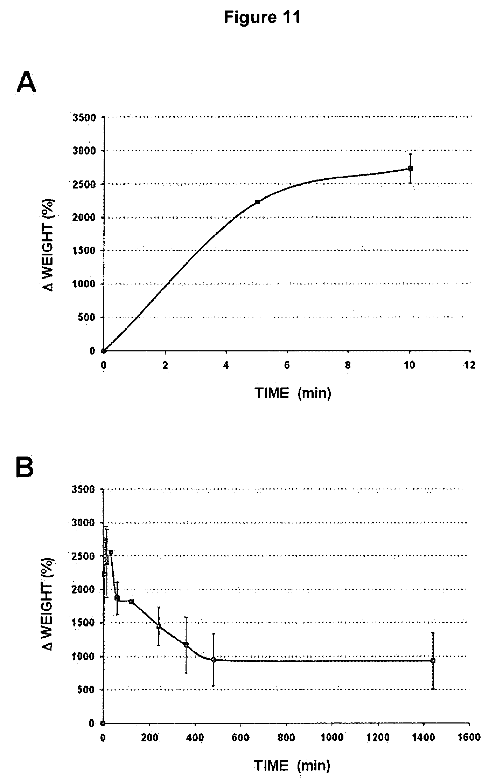

Likewise, in Figure 11, with respect to matrices obtained with 6% $HELP_8$ concentration, A shows the first part of the curve regarding the attainment of the maximum absorption, which occurred after 10 minutes.

Subsequently, as shown in B, a rapid weight loss occurs up to a plateau around a value of 500% weight variation after 24 hours testing, which is indicative of a meaningful loss of material cohesion.

*Example 7: characterization by Fourier transform infrared spectroscopy of 3D matrices obtained by cross-linking of $HELP_8$ polypeptide with SEQ ID NO:1 (n= 8)*

$HELP_8$ matrices cross-linked with transglutaminase, lyophilized (dried until reaching a constant weight), when analyzed by Fourier transform infrared spectroscopy in attenuated total reflection mode (ATR-FTIR) show a different spectrum compared to $HELP_8$ not-treated with the enzyme.

An index of the extent of cross-linking can be expressed as the ratio between the band attributed to stretching of the C-N bond (at 1090-1045 $cm^{-1}$) of the primary amino group and the band attributed to stretching of the C-H bond (at 2940-2840 $cm^{-1}$), taken as a reference. As expected, following the cross-linking reaction via enzyme action, the value of such ratio decreases by one order of magnitude upon transition from the linear HELP polypeptide to the $HELP_8$ lattice (see Table 1).

| Table 1. Values of the ratio between *stretching* ($\nu$) of the C-N bond and $\nu$ of the C-H bond, for $HELP_8$ not-treated with the enzyme and for cross-linked $HELP_8$ matrices. | |
| --- | --- |
| | $\nu(C-N)/\nu(C-H)$ |
| $HELP_8$ | 0.458 |
| 3% matrix | 0.031 |
| 5% matrix | 0.027 |
| 6% matrix | 0.020 |

*Example 8: cell culture on hydrogel supports obtained by cross-linking of $HELP_8$ polypeptide with SEQ ID NO:1 (n=8)*

The 5% $HELP_8$ matrix obtained according to the conditions described was tested as support for the growth of cell lines. All tests were done in triplicate.

The $HELP_8$ polypeptide was sterilized by filtration and then lyophilised. The material thus obtained was re-dissolved in an appropriate volume of 10mMTris/HCl pH8 in order to obtain a 5% solution which was subjected to cross-linking inside the wells of a sterile micro-titre plate, in presence of filter-sterilized transglutaminase. After preparation, the matrix can be left immersed in saline or culture medium is order to facilitate enzyme diffusion, hence removal of the enzyme prior to cell seeding.

All wells were seeded in the same way, and in parallel, with 5000 epG2 cells and monitored for 10 days without changing the culture medium consisting of complete DMEM supplemented with 10% fetal calf serum, penicillin and streptomycin. Cell profiferation analysis was done at prefixed times; in particular the proliferation profile was monitored at 24, 48, 72, 96, 168, 240 hours, comparing the growth on 5% $HELP_8$ and on a standard treated plastic support.

At the indicated times after seeding, cell proliferation was assessed by colorimetric assay with WST-1 (Water Soluble Tetrazolium -1, Roche Applied Science).

The result obtained in this type of test is proportional to the number of cells that is metabolically active under the experimental conditions used. The molecule, which absorbs very little at 450 nm, is modified by cells into a compound which has an adsorption peak precisely at this wavelength and is released in the surrounding environment.

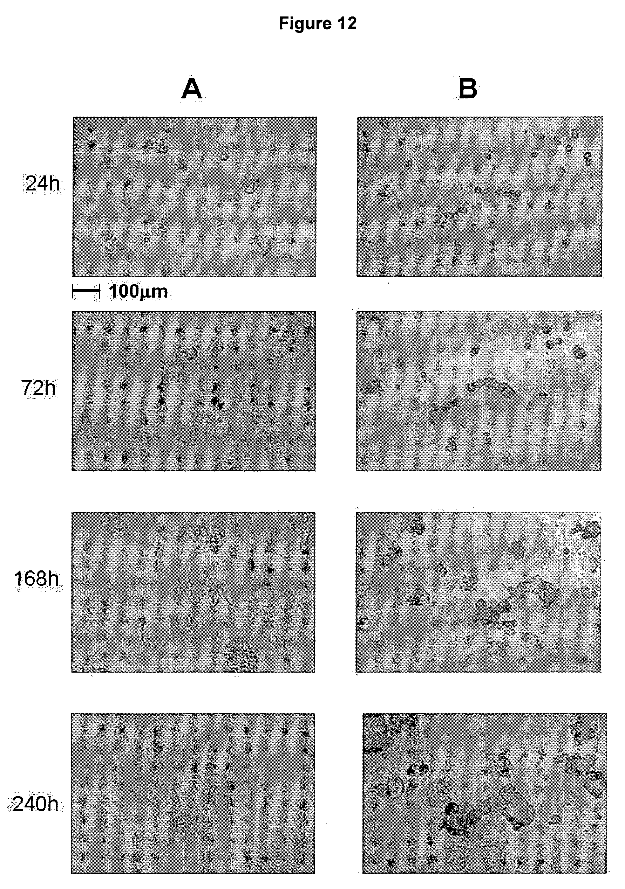

In parallel, one field of cells grown on standard treated plastic and one field of cells grown on $HELP_8$ have been selected in order to document their morphology. As highlighted in figures 12 an 13, cell growth on the matrix shows a slower start, as already reported in the literature for other three-dimensional supports. It is clearly observed that cells grown on treated standard plastic reached confluence within the days, while those on $HELP_8$ matrix were entering the exponential phase. As described in the literature, proliferation ont the matrix involves growth of these cells as ''islets'' and a more rounded cellular morphology compared to cells grown on standard treated plastic. Therefore the support is not toxic.

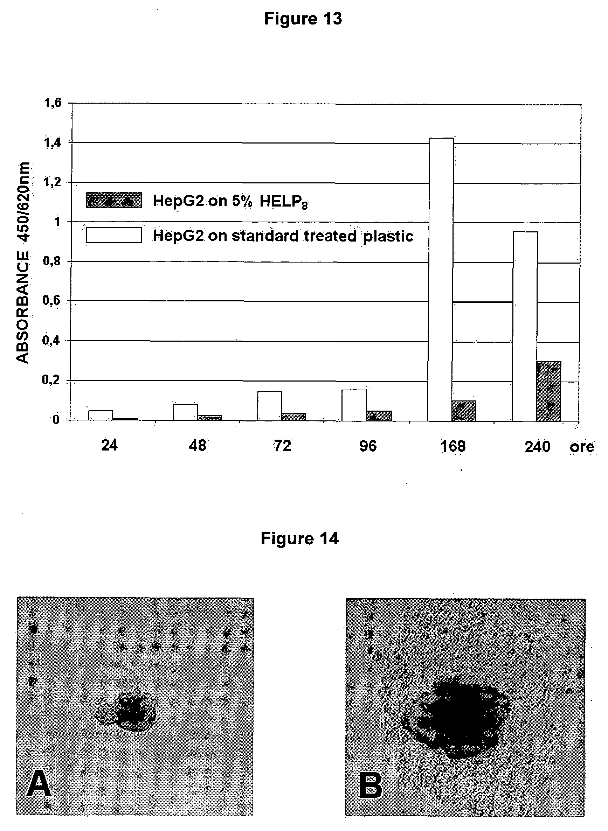

It was therefore interesting to evaluate the extent of viability of HepG2 cells that were left to proliferate on HELP8: One of the ''islet-shaped'' colonies that formed on the matrix was taken after one week and transferred to the standard treated plastic surface. Figure 14 shows the appearance of a colony that was just transferred to standard treated plastic (A) and the appearance of the same after 12 days of cultre (B), without further changes of culture medium. Cells in contact with the standard treated plastic begin to re-adhere and spread around as a halo, thus showing good viability.

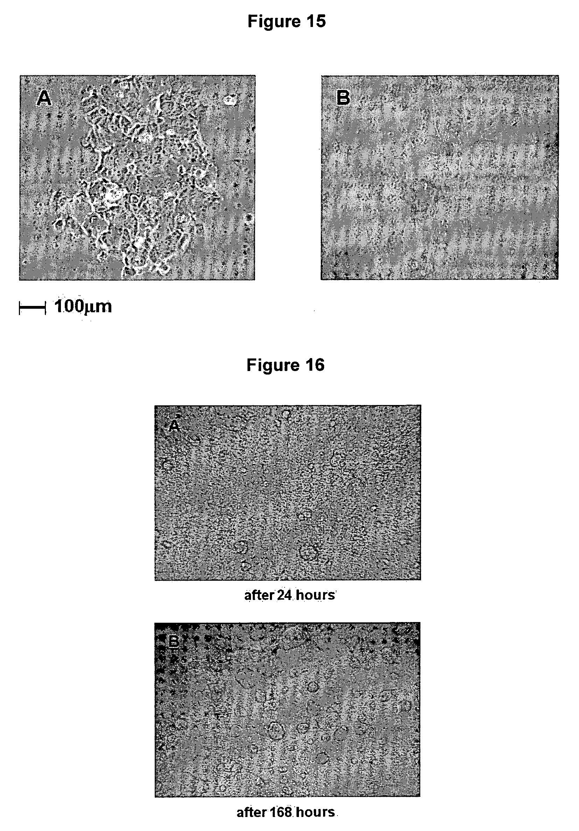

The growth assays were also carried out with another cell line, also of human origin, reportedly known to grow in other three-dimensional matrices. Like HepG2 cells, MCF-7 cells proved well capable of growing on 5% HELP$_8$ matrix, as shown in figure 15. Also this cell line, under the same conditions with respect to growth medium, showed a good degree of proliferation and a more rounded morphology compared to the morphology which develops on standard treated plastic.

*Example 9: encapsulation of cells in hydrogels obtained by cross-linking the HELP$_8$ polypeptide with SEQ ID NO:1 (n=8)*

These cells were used for the encapsulation test; for this purpose, cells collected as pellet by centrifugation were re-suspended directly in 3% HELP$_8$ solution containing sterile 10mM Tris/HCl and 150mM NaCl, followed by addition of transglutaminase enzyme as described above, the whole was mixed and immediately transferred to the wells and left for 30 minutes at room temperature. After cross-linking, culture medium was added and cells were observed under phase contrast microscope. As highlighted in figure 16, not only encapsulated cells showed survival ability but also a good proliferative capacity, indicating that the direct treatment with the enzyme, in presence of cells, does not undermine cellular viability.

The growth assays were also carried out with another cell line, also of human origin, reportedly known to grow in other three-dimensional matrices. Like HepG2 cells, MCF-7 cells proved well capable of growing on 5% HELP$_8$ matrix, as shown in figure 15. Also this cell line, under the same conditions with respect to growth medium, showed a good degree of proliferation and a more rounded morphology compared to the morphology which develops on standard treated plastic.

*Example 9: encapsulation of cells in hydrogels obtained by cross-linking the HELP$_8$ polypeptide with SEQIDNO:1 (n=8)*

These cells were used for the encapsulation test; for this purpose, cells collected as pellet by centrifugation were re-suspended directly in 3% HELP$_8$ solution containing sterile 10mM Tris/HCl and 150mM NaCl, followed by addition of transglutaminase enzyme as described above, the whole was mixed and immediately transferred to the wells and left for 30 minutes at room temperature. After cross-linking, culture medium was added and cells were observed under phase contrast microscope. As highlighted in figure 16, not only encapsulated cells showed survival ability but also a good proliferative capacity, indicating that the direct treatment with the enzyme, in presence of cells, does not undermine cellular viability.

## Claims

1. 3D matrices of human elastin-like polypeptides **characterized in that** they are obtainable by transglutaminase enzymatic cross-linking of at least one human elastin-like polypeptide having sequence MRGSHHHHHHG-SAA(AAAAAAKAAAKAAQFGLVPGVGVAPGVGVAPGV GVAPGVGLAPGVGVAPGVGVAPGVGVAPGIAP)$_n$GV (SEQ ID NO:1) where n is an integer comprised between 4 and 12.

2. 3D matrices of human elastin-like polypeptides according to claim 1, wherein in the SEQ ID NO:1 n is an integer comprised between 6 and 10.

3. 3D matrices of human elastin-like polypeptides according to claim 1, wherein in the SEQ ID NO:1 n is equal to 8.

4. 3D matrices of human elastin-like polypeptides according to claim 1, wherein the cross-linking is obtainable by means of at least the steps of:

   - preparing an aqueous solution buffered at pH 8 of a human elastin-like polypeptide of SEQ ID NO:1 at a concentration selected in the range from 3% to 6% w/v;
   - treating the aqueous solution of the polypeptide with an aqueous solution of transglutaminase at a concentration of 2 mg/ml and at a temperature comprised between 20°C and 25°C.

5. 3D matrices of human elastin-like polypeptides according to claim 4, wherein the aqueous solutions of the human elastin-like polypeptide of SEQ ID NO:1 are buffered with 10mM TRIS/HCl.

6. 3D matrices of human elastin-like polypeptides according to claim 4, wherein the aqueous solutions have an ionic strength comprised between 0 and 150 mM.

7. 3D matrices of human elastin-like polypeptides according to claim 3, **characterized by** values of the ratio of *stretching* (ν) of the C-N bond (at 1090-1045 cm$^{-1}$) and *stretching* (ν) of the C-H bond (at 2940-2840 cm$^{-1}$) (ν (C-N)/ν (C-H)) comprised between 0.031 and 0.020.

8. 3D matrices of human elastin-like polypeptides according to one of the preceding claims comprising cells and/or pharmacologically or biologically active molecules.

9. Method for preparation of 3D matrices of human elastin-like polypeptides **characterized by** being obtainable by enzymatic cross-linking of at least one human elastin-like polypeptide having sequence MRGSHHHHHHGSAA(AAAAAAKAAAKAAQFGLVPGVGVAPGVGVAPGV GVAPGVGLAPGVGVAPGVGVAPGVGVAPGIAP)nGV (SEQ ID NO:1) where n is an integer comprised between 4 and 12 comprising at least the steps of :

   - preparing an aqueous solution buffered at pH 8 of the human elastin-like polypeptide of SEQ ID NO:1 at a concentration selected in the range from 3% to 6% w/v;
   - treating the aqueous solutions of the polypeptide with an aqueous solution of transglutaminase at a concentration of 2 mg/ml and at a temperature comprised between 20°C and 25°C.

10. Method for preparation of 3D matrices of human elastin-like polypeptides according to claim 9, wherein in SEQ_ID_NO:1 n is an integer comprised between 6 and 10.

11. Method for preparation of 3D matrices of human elastin-like polypeptides according to claim 9, wherein in SEQ ID NO:1 n is equal to 8.

**12.** Method for preparation of 3D matrices of human elastin-like polypeptides according to claim 9, wherein the aqueous solutions of the human elastin-like polypeptide of SEQ ID NO:1 are buffered with 10mM TRIS/HCl.

**13.** Method for preparation of 3D matrices of human elastin-like polypeptides according to claim 9, wherein the aqueous solutions have an ionic strength comprised between 0 and 150 mM.

**14.** Method for preparation of 3D matrices of human elastin-like polypeptides according to claim 9, wherein cells in suspension and/or pharmacologically or biologically active molecules are also added to the aqueous solutions.

**15.** Method for preparation of 3D matrices of human elastin-like polypeptides according to claim 9, further comprising a step of removal of the buffering agent and/or the enzyme by diffusion in aqueous medium.

**16.** Use of 3D matrices of human elastin-like polypeptides, as defined in one of the claims from 1 to 8, in the biomedical field for research purposes.

**17.** Use of 3D matrices of human elastin-like polypeptides, as defined in one of the claims from 1 to 8, in the biomedical field for tissue engineering.

**Patentansprüche**

**1.** 3D-Matrizes von humanen elastinähnlichen Polypeptiden, **dadurch gekennzeichnet, dass** sie durch eine enzymatische Vernetzung mit Transglutaminase von mindestens einem elastinähnlichen Polypeptid mit der Sequenz MRGSHHHHHHGSAA(AAAAAAKAAAKAAQFGLVPGVGVAPGVGVAPGVGVAPGV GLAPGVGVAPGVG-VAPGVGVAPGIAP)nGV (SEQ ID NO: 1) erhalten werden können, wobei n eine ganze Zahl im Bereich zwischen 4 und 12 ist.

**2.** 3D-Matrizes von humanen elastinähnlichen Polypeptiden gemäß Anspruch 1, wobei in der SEQ ID NO: 1 n eine ganze Zahl im Bereich zwischen 6 und 10 ist.

**3.** 3D-Matrizes von humanen elastinähnlichen Polypeptiden gemäß Anspruch 1, wobei in der SEQ ID NO: 1 n gleich 8 ist.

**4.** 3D-Matrizes von humanen elastinähnlichen Polypeptiden gemäß Anspruch 1, wobei die Vernetzung mittels mindestens einem der folgenden Schritte erhalten werden kann:

Herstellen einer wässrigen, bei pH 8 gepufferten Lösung von humanem elastinähnlichen Polypeptid der SEQ ID NO: 1 mit einer Konzentration ausgewählt aus dem Bereich von 3 Gew.-% bis 6 Gew.-%;
Behandeln der wässrigen Lösung des Polypeptids mit einer wässrigen Lösung von Transglutaminase mit einer Konzentration von 2 mg/ml und bei einer Temperatur im Bereich zwischen 20 °C und 25 °C.

**5.** 3D-Matrizes von humanen elastinähnlichen Polypeptiden gemäß Anspruch 4, wobei die wässrigen Lösungen von humanem elastinähnlichen Polypeptid von SEQ ID NO: 1 mit 10 mM TRIS/HCl gepuffert sind.

**6.** 3D-Matrizes von humanen elastinähnlichen Polypeptiden gemäß Anspruch 4, wobei die wässrigen Lösungen eine Ionenstärke im Bereich zwischen 0 und 150 mM aufweisen.

**7.** 3D-Matrizes von humanen elastinähnlichen Polypeptiden gemäß Anspruch 3, **dadurch gekennzeichnet, dass** die Werte des Verhältnisses von *Streckschwingung* ($\nu$) der C-N-Bindung (bei 1090-1045 cm$^{-1}$) und der *Streckschwingung* ($\nu$) der C-H-Bindung (bei 2940-2840 cm$^{-1}$) ($\nu$ (C-N)/$\nu$ (C-H)) in einem Bereich zwischen 0,031 und 0,020 liegen.

**8.** 3D-Matrizes von humanen elastinähnlichen Polypeptiden gemäß einem der vorhergehenden Ansprüche, umfassend Zellen und/oder pharmakologisch oder biologisch aktive Moleküle.

**9.** Verfahren zur Herstellung von 3D-Matrizes von humanen elastinähnlichen Polypeptiden, **dadurch gekennzeichnet, dass** sie durch eine enzymatische Vernetzung von mindestens einem elastinähnlichen Polypeptid mit der Sequenz MRGSHHHHHHGSAA(AAAAAAKAAAKAAQFGLVPGVGVAPGVGVAPGVGVAPGV GLAPGVGVAPGVG-VAPGVGVAPGIAP)nGV (SEQ ID NO: 1) erhalten werden können, wobei n eine ganze Zahl im Bereich zwischen 4 und 12 ist, umfassend mindestens die folgenden Schritte:

Herstellen einer wässrigen, bei pH 8 gepufferten Lösung von humanem elastinähnlichen Polypeptid der SEQ ID NO: 1 mit einer Konzentration ausgewählt aus dem Bereich von 3 Gew.-% bis 6 Gew.-%;
Behandeln der wässrigen Lösungen des Polypeptids mit einer wässrigen Lösung von Transglutaminase mit einer Konzentration von 2 mg/ml und bei einer Temperatur im Bereich zwischen 20 °C und 25 °C.

**10.** Verfahren zur Herstellung von 3D-Matrizes von humanen elastinähnlichen Polypeptiden gemäß Anspruch 9, wobei in der SEQ ID NO: 1 n eine ganze Zahl im Bereich zwischen 6 und 10 ist.

**11.** Verfahren zur Herstellung von 3D-Matrizes von humanen elastinähnlichen Polypeptiden gemäß Anspruch 9, wobei in der SEQ ID NO: 1 n gleich 8 ist.

**12.** Verfahren zur Herstellung von 3D-Matrizes von humanen elastinähnlichen Polypeptiden gemäß Anspruch 9, wobei die wässrigen Lösungen von humanem elastinähnlichen Polypeptid von SEQ ID NO: 1 mit 10 mM TRIS/HCl gepuffert sind.

**13.** Verfahren zur Herstellung von 3D-Matrizes von humanen elastinähnlichen Polypeptiden gemäß Anspruch 9, wobei die wässrigen Lösungen eine Ionenstärke im Bereich zwischen 0 und 150 mM aufweisen.

**14.** Verfahren zur Herstellung von 3D-Matrizes von humanen elastinähnlichen Polypeptiden gemäß Anspruch 9, wobei auch Zellen in Suspension und/oder pharmakologisch oder biologisch aktive Moleküle zu den wässrigen Lösungen gegeben werden.

**15.** Verfahren zur Herstellung von 3D-Matrizes von humanen elastinähnlichen Polypeptiden gemäß Anspruch 9, des Weiteren umfassend einen Schritt des Entfernens des Puffermittels und/oder des Enzyms durch Diffusion in wässrigem Medium.

**16.** Verwendung von 3D-Matrizes von humanen elastinähnlichen Polypeptiden definiert gemäß einem der Ansprüche von 1 bis 8 im biomedizinischen Bereich für Forschungszwecke.

**17.** Verwendung von 3D-Matrizes von humanen elastinähnlichen Polypeptiden definiert gemäß einem der Ansprüche von 1 bis 8 im biomedizinischen Bereich zur künstlichen Gewebeherstellung (Tissue Engineering).

**Revendications**

**1.** Matrices en 3D de polypeptides de type élastine humaine **caractérisées en ce qu'**elles peuvent être obtenues par réticulation enzymatique avec une transglutaminase d'au moins un polypeptide de type élastine humaine ayant la séquence MRGSHHHHHHGSAA (AAAAAAKAAAKAAQFGLVPGVGVAPGVGVAPGVGVAPGVGLAPGVGVAPGVGVA PGVGVAPGIAP)$_n$GV (SEQ ID NO : 1) où n est un nombre entier compris entre 4 et 12.

**2.** Matrices en 3D de polypeptides de type élastine humaine selon la revendication 1, dans lesquelles dans la SEQ ID NO : 1 n est un nombre entier compris entre 6 et 10.

**3.** Matrices en 3D de polypeptides de type élastine humaine selon la revendication 1, dans lesquelles dans la SEQ ID NO : 1 n est égal à 8.

**4.** Matrices en 3D de polypeptides de type élastine humaine selon la revendication 1, dans lesquelles la réticulation peut être obtenue au moyen d'au moins les étapes de :

- préparation d'une solution aqueuse tamponnée à pH 8 d'un polypeptide de type élastine humaine de SEQ ID NO : 1 à une concentration choisie dans la plage de 3 % à 6 % p/v ;
- traitement de la solution aqueuse du polypeptide avec une solution aqueuse de transglutaminase à une concentration de 2 mg/ml et à une température comprise entre 20 °C et 25 °C.

**5.** Matrices en 3D de polypeptides de type élastine humaine selon la revendication 4, dans lesquelles les solutions aqueuses des polypeptides de type élastine humaine de SEQ ID NO : 1 sont tamponnées avec 10 mM de TRIS/HCl.

**6.** Matrices en 3D de polypeptides de type élastine humaine selon la revendication 4, dans lesquelles les solutions aqueuses ont une force ionique comprise entre 0 et 150 mM.

**7.** Matrices en 3D de polypeptides de type élastine humaine selon la revendication 3, **caractérisées par** des valeurs du rapport de l'étirement ($\nu$) de la liaison C-N (à 1090-1045 cm$^{-1}$) et de l'étirement ($\nu$) de la liaison C-H (à 2940-2840 cm$^{-1}$) ($\nu$ (C-N)/$\nu$ (C-H)) comprises entre 0,031 et 0,020.

**8.** Matrices en 3D de polypeptides de type élastine humaine selon l'une quelconque des revendications précédentes comprenant des cellules et/ou des molécules pharmacologiquement ou biologiquement actives.

**9.** Procédé pour la préparation de matrices en 3D de polypeptides de type élastine humaine **caractérisées en ce qu'**elles peuvent être obtenues par réticulation enzymatique d'au moins un polypeptide de type élastine humaine ayant la séquence MRGSHHHHHHGSAA(AAAAAAKAAAKAAQ FGLVPGVGVAPGVGVAPGVGVAPGVGLAPGVGVAPGVGVAPGVGVAPGIAP)$_n$GV (SEQ ID NO : 1) où n est un nombre entier compris entre 4 et 12 comprenant au moins les étapes de :

- préparation d'une solution aqueuse tamponnée à pH 8 du polypeptide de type élastine humaine de SEQ ID NO : 1 à une concentration choisie dans la plage de 3 % à 6 % p/v ;
- traitement des solutions aqueuses du polypeptide avec une solution aqueuse de transglutaminase à une concentration de 2 mg/ml et à une température comprise entre 20 °C et 25 °C.

**10.** Procédé pour la préparation de matrices en 3D de polypeptides de type élastine humaine selon la revendication 9, dans lequel dans SEQ ID NO : 1 n est un nombre entier compris entre 6 et 10.

**11.** Procédé pour la préparation de matrices en 3D de polypeptides de type élastine humaine selon la revendication 9, dans lequel dans SEQ ID NO : 1 n est égal à 8.

**12.** Procédé pour la préparation de matrices en 3D de polypeptides de type élastine humaine selon la revendication 9, dans lequel les solutions aqueuses du polypeptide de type élastine humaine de SEQ ID NO : 1 sont tamponnées avec 10 mM de TRIS/HCl.

**13.** Procédé pour la préparation de matrices en 3D de polypeptides de type élastine humaine selon la revendication 9, dans lequel les solutions aqueuses ont une force ionique comprise entre 0 et 150 mM.

**14.** Procédé pour la préparation de matrices en 3D de polypeptides de type élastine humaine selon la revendication 9, dans lequel des cellules en suspension et/ou des molécules pharmacologiquement ou biologiquement actives sont également ajoutées aux solutions aqueuses.

**15.** Procédé pour la préparation de matrices en 3D de polypeptides de type élastine humaine selon la revendication 9, comprenant en outre une étape d'élimination de l'agent tampon et/ou de l'enzyme par diffusion en milieu aqueux.

**16.** Utilisation de matrices en 3D de polypeptides de type élastine humaine, telles que définies dans l'une des revendications de 1 à 8, dans le domaine biomédical pour des objectifs de recherche.

**17.** Utilisation de matrices en 3D de polypeptides de type élastine humaine, telles que définies dans l'une des revendications de 1 à 8, dans le domaine biomédical pour le génie tissulaire.

# Figure 1

## Figure 2

## Figure 3

5% HELP$_8$          5% HELP$_8$-150mM NaCl

# Figure 4

## Figure 5

3%

A

5%

B

⊢━━┤

100 μm

6%

C

## Figure 6

**A**

**B**

Figure 7

Figure 8

A

B

Figure 9

## Figure 10

A

B

## Figure 11

**A**

**B**

# Figure 12

**A**    **B**

24h

|—| **100μm**

72h

168h

240h

## Figure 13

## Figure 14

# Figure 15

100μm

# Figure 16

after 24 hours

after 168 hours

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4474851 A
- US 4500700 A
- US 4870055 A
- US 5250516 A
- US 4589882 A
- US 20050255554 A1, Chilkoti, A.
- US 20030166846 A1, Keeley, F. W.
- US 20080312156 A1
- IT PD20090092 A

**Non-patent literature cited in the description**

- **WISE, S.G. ; WEISS, A.S.** *Int. J. Biochem. Cell Biol.,* 2009, vol. 41, 494-497
- **VRHOVSKI, B. ; WEISS, A.S.** *Eur. J. Biochem.,* 1998, vol. 258, 1-18
- **URRY, D.W. et al.** *Philos. Trans. R Soc. Lond. B Biol. Sci,* 2002, vol. 357, 169-184
- **BELLINGHAM C. M. et al.** *Biochim. Biophys. Acta,* 2001, vol. 1550, 6-19
- **ANNABI, N. et al.** *Biomaterials,* 2009, vol. 30, 1-7
- **MITHIEUX S. M. et al.** *Biomaterials,* 2004, vol. 25, 4921-4927
- **MITHIEUX, S.M. et al.** *Biomaterials,* 2009, vol. 30, 431-435
- **CARLSON, K. et al.** *Biomacromolecules,* 2003, vol. 4, 572-580
- **LIM, D.W.** *Biomacromolecules,* 2007, vol. 8, 1463-1470
- **MCHALE, M. K. et al.** *Tissue Eng.,* 2005, vol. 11, 1768-1779
- **LAO, U. L. et al.** *Biomacromolecules,* 2007, vol. 8, 3736-3739
- **DINERMAN, A. A. et al.** *Biomaterials,* 2002, vol. 23, 4203-4210
- **BELLINGHAM, C. M. et al.** *Biopolymers,* 2003, vol. 70, 445-55
- **VIETH, S. et al.** *Biopolymers,* 2007, vol. 85, 199-206
- **SROKOWSKI, E.M et al.** *J. Biomater. Sci. Polym. Ed.,* 2008, vol. 19, 785-799
- **GARCIA Y. et al.** *Tissue Eng. Part A.,* 2009, vol. 15, 887-899
- **HU BI- HUANG ; MESSERSMITH P.** *J. Am. Chem. Soc.,* 2003, vol. 125, 14298-14299
- **BANDIERA A. et al.** *Biotechnol. Appl. Biochem.,* 2005, vol. 42, 247-256
- **BANDIERA A. et al.** *Bioengineering Conference, 2009 IEEE 35th Annual Northeast,* 03 April 2009
- **BANDIERA A et al.** *Biotechnol. Appl. Biochem.,* 2005, vol. 42, 247-256

SEQUENCE LISTING

<110> Università degli Studi di Trieste

<120> 3D matrices of human-like elastine polypeptides and method of preparation thereof

<130> 9612 PTWO

<150> IT PD2009A000092
<151> 2009-04-16

<160> 5

<170> PatentIn version 3.5

<210> 1
<211> 81
<212> PRT
<213> Artificial

<220>
<223> chimeric sequence

<220>

<221> MISC_FEATURE
<222> (1)..(81)
<223> Base unity polypeptide containing the repeated sequence IDN2 n folds between Ala in position 14 and Gly in position 80

<400> 1

```
        Met Arg Gly Ser His His His His His His Gly Ser Ala Ala Ala Ala
        1               5                   10                  15


        Ala Ala Ala Ala Lys Ala Ala Ala Lys Ala Ala Gln Phe Gly Leu Val
                    20                  25                  30


        Pro Gly Val Gly Val Ala Pro Gly Val Gly Val Ala Pro Gly Val Gly
                    35                  40                  45


        Val Ala Pro Gly Val Gly Leu Ala Pro Gly Val Gly Val Ala Pro Gly
                    50                  55                  60


        Val Gly Val Ala Pro Gly Val Gly Val Ala Pro Gly Ile Ala Pro Gly
            65                  70                  75                  80


        Val
```

<210> 2
<211> 68
<212> PRT
<213> Artificial Sequence

<220>
<223> repeated monomer

<220>
<221> MISC_FEATURE
<222> (1)..(68)
<223> monomeric unit repeated in SEQ IDN1

<400> 2

```
Ala Ala Ala Ala Ala Ala Lys Ala Ala Ala Lys Ala Ala Gln Phe Gly
1               5                   10              15

Leu Val Pro Gly Val Gly Val Ala Pro Gly Val Gly Val Ala Pro Gly
            20              25              30

Val Gly Val Ala Pro Gly Val Gly Leu Ala Pro Gly Val Gly Val Ala
            35              40              45

Pro Gly Val Gly Val Ala Pro Gly Val Gly Val Ala Ala Arg Thr Pro
            50              55              60

Gly Ile Ala Pro
            65
```

<210> 3
<211> 15
<212> PRT
<213> Homo sapiens

<400> 3

```
Ala Ala Ala Ala Ala Ala Lys Ala Ala Ala Lys Ala Ala Gln Phe
1               5                   10              15
```

<210> 4
<211> 50
<212> PRT
<213> Artificial Sequence

<220>
<223> elastin-like domain

<400> 4

```
Gly Leu Val Pro Gly Val Gly Val Ala Pro Gly Val Gly Val Ala Pro
1               5                   10              15

Gly Val Gly Val Ala Pro Gly Val Gly Leu Ala Pro Gly Val Gly Val
            20              25              30

Ala Pro Gly Val Gly Val Ala Pro Gly Val Gly Val Ala Pro Gly Ile
            35              40              45

Ala Pro
    50
```

<210> 5
<211> 6
<212> PRT
<213> Homo sapiens

<220>
<221> DOMAIN
<222> (1)..(6)

<400> 5

```
Val Ala Pro Gly Val Gly
1                   5
```